# EUROPÄISCHE PATENTSCHRIFT

(11) **EP 2 058 319 B1**
(45) Veröffentlichungstag und Bekanntmachung des Hinweises auf die Patenterteilung: **17.08.2011**
(21) Anmeldenummer: 09002406.8
(22) Anmeldetag: 17.03.2003
(51) Int. Cl.: C07F 9/50

(54) **Verwendung von Perfluoralkylphosphinen als Perfluoralkylierungsreagenzien**
Use of perfluoralkylphosphines as perfluoroalkylation reagents
Utilisation de perfluoro-alkylphosphines en tant que réactifs de perfluoroalkylation

(30) Priorität: 18.04.2002 DE 10216998
(43) Veröffentlichungstag der Anmeldung: 13.05.2009
(62) Teilanmeldung aus: 03712029.2
(73) Patentinhaber: Merck Patent GmbH, 64293 Darmstadt (DE)
(72) Erfinder: Welz-Biermann, Urs, Prof. Dr., Dalian 116021 (CN); Ignatyev, Nikolai, (Mikola) Dr., 47058 Duisburg (DE); Weiden, Michael, Dr., 64285 Darmstadt (DE); Schmidt, Michael, Dr., 64342 Seeheim-Jugenheim (DE); Heider, Udo, Dr., 64579 Gernsheim (DE); Miller, Alexej, Dr., 91052 Erlangen (DE); Willner, Helge, Prof. Dr., 45481 Mülheim/Ruhr (DE); Sartori, Peter, Prof. Dr., 86919 Utting (DE)

(56) Entgegenhaltungen:
- FR-A- 2 827 285
- US-A- 4 701 569
- US-A- 6 096 926
- KAMPA J J: "The synthesis of tris(perfluoroalkyl) phosphanes" ANGEWANDTE CHEMIE. INTERNATIONAL EDITION, VERLAG CHEMIE. WEINHEIM, DE, Bd. 34, Nr. 11, 1995, Seiten 1241-1244, XP002125737 ISSN: 0570-0833

## Beschreibung

Die vorliegende Erfindung betrifft die Verwendung von Perfluoralkylphosphinen als Perfluoralkylierungreagenzien.

Perfluoralkylphosphine sind an sich bekannt. Nach dem in der Literatur beschriebenen Verfahren werden einige Verbindungen durch Reduktion entsprechender Difluortris(perfluoralkyl)phosphorane mit P[Si(CH₃)₃]₃ gewonnen (J.J. Kampa et al., Angew. Chem., 107, Nr. 11 (1995), Seiten 1334-1337). Nachteilig bei diesem Verfahren ist insbesondere die sehr geringe Ausbeute der Perfluoralkylphosphine sowie die relativ aufwendige Herstellung des Reduktionsmittels P[Si(CH₃)₃]₃.

Die Aufgabe der vorliegenden Erfindung bestand daher darin, eine neue Verwendung von Perfluoralkylphosphinen zu finden.

Diese Aufgabe wurde durch die erfindungsgemäße Verwendung gelöst.

Die Herstellung von Fluor(perfluoralkyl)phosphosphoranen kann nach üblichen, dem Fachmann bekannten Methoden erfolgen. Vorzugsweise werden diese Verbindungen durch elektrochemische Fluorierung geeigneter Ausgangsverbindungen hergestellt, wie in V.Ya. Semenii et al., Zh. Obshch.Khim., 55, Nr. 12 (1985), Seiten 2716-2720; N. Igantiev, P. Sartori, J. of Fluorine Chem., 103 (2000), Seiten 57-61 sowie der WO 00/21969 beschrieben. Die

Zur Herstellung von Perfluoralkylphosphinen können ein oder mehrere Hyrid-Ionen-Donatoren, d.h. Verbindungen, die in Lage sind, ein oder mehrere Hyrid-lonen (H') abzugeben, jeweils einzeln oder in Kombination zum Einsatz kommen, wobei der Einsatz jeweils nur eines Hydrid-Ionen-Donators bevorzugt ist.

Vorzugsweise ist der Hydrid-Ionen-Donator ausgewählt aus der Gruppe bestehend aus Hydrosilanen, Alkylhydrosilanen, Metallhydriden, Borhydriden und Hydroboraten.

Sofern als Hydrid-Ionen-Donator ein Alkylhydrosilan zum Einsatz kommt, ist dies bevorzugt Triethylsilan oder Tripropylsilan.

Kommt als Hydrid-Ionen-Donator ein Borhydrid zum Einsatz, ist dies bevorzugt Natriumborhydrid.

In einer bevorzugten Ausführungsform des Verfahrens wird wenigstens eine Fluor(perfluoralkyl)phosphoranverbindung der allgemeinen Formel I

(CₙF₂ₙ₊₁)ₘPF₅₋ₘ

mit 1 ≤ n ≤ 8, vorzugsweise 1 ≤ n ≤ 4 und m jeweils gleich 1, 2 oder 3 mit einem Hydrid-Ionen-Donator umgesetzt.

Besonders bevorzugte Fluor(perfluoralkyl)phosphoranverbindungen können ausgewählt werden aus der Gruppe bestehend aus Difluortris(pentafluorethyl)phosphoran, Difluortris(n-nonafluorbutyl)phosphoran und Difluortris(n-heptafluorpropyl)phosphoran, Trifluorbis(n-nonafluorbutyl)phosphoran.

Die Umsetzung wenigstens einer Fluor(perfluoralkyl)phosphoranverbindung kann sowohl in einem geeigneten Reaktionsmedium als auch ohne Gegenwart eines Reaktionsmediums durchgeführt werden. Bevorzugt erfolgt die Umsetzung ohne Reaktionsmedium, da hierdurch die Umweltbilanz des Verfahrens verbessert und die Kosten reduziert werden.

Die zum Einsatz kommenden Hydrid-Ionen-Donatoren werden vorzugsweise im Überschuß, jeweils bezogen auf die eingesetzte Menge an Fluor(perfluoralkyl)phosphoran, eingesetzt, um eine vollständige Umsetzung zu den gewünschten Perfluoralkylphosphinen zu gewährleisten. Ebenfalls bevorzugt können die Hydrid-lonen-Donatoren äquimolar, jeweils bezogen auf die eingesetzte Menge an Fluor(perfluoralkyl)phosphoran, eingesetzt werden.

Die Temperatur während der Umsetzung sowie deren Dauer können beispielsweise in Abhängigkeit voneinander sowie in Abhängigkeit von dem eingesetzten Fluor(perfluoralkyl)phosphoran und den jeweils gewählten Ansatzgrößen über einen weiten Bereich variieren. Die jeweils optimale Wahl der Parameter kann vom Fachmann durch einfache Vorversuche ermittelt werden.

In einer bevorzugten Ausführungsform des Verfahrens wird während der Umsetzung des Fluor(perfluoralkyl)phosphorans unter Rückfluß erhitzt.

Die Dauer der Umsetzung beträgt vorzugsweise 0,5 bis 20 Stunden. Sofern Triethylsilan als Hydrid-Ionen-Donator verwendet wird, beträgt die Dauer besonders bevorzugt 3 bis 15 Stunden, für Natriumborhydrid besonders bevorzugt 1 bis 3 Stunden.

An die Herstellung eines oder mehrerer Perfluoralkylphosphine nach dem beschriebenen Verfahren kann sich, falls erforderlich, eine Reinigung dieser Verbindungen nach üblichen, dem Fachmann bekannten Methoden anschließen.

In einer bevorzugten Ausführungsform des Verfahrens können die hergestellten Perfluoralkylphosphine durch ein- oder mehrfache Destillation, gegebenenfalls unter vermindertem Druck und/oder ggf. unter Inertgasatmosphäre gereinigt und ggf. isoliert werden.

Das beschriebeneVerfahren zur Herstellung von Perfluoralkylphosphinen ermöglicht die kostengünstige Herstellung dieser Verbindungen in großer Ausbeute und in hohen Reinheiten. Es zeichnet sich ferner dadurch aus, daß die zum Einsatz kommenden Hydrid-Ionen-Donatoren käuflich erhältliche, kostengünstige Verbindungen darstellen, die auch im großtechnischen Maßstab problemlos gehandhabt werden können. Die als Ausgangsverbindungen eingesetzten Fluor(perfluoralkyl)phosphorane können durch elektrochemische Fluorierung ebenfalls kostengünstig hergestellt werden.

Vorteilhaft ist weiterhin, daß das Verfahren lösungsmittelfrei durchgeführt werden kann, was die Herstellungskosten der Perfluoralkylphosphine weiter senkt und die Umweltbilanz des Verfahrens verbessert.

Überraschenderweise wurde auch gefunden, daß sich Tris(perfluoralkyl)phosphine zur Perfluoralkylierung chemischer Substrate eignen.

Die Perfluoralkylierung stellt ein wichtiges Verfahren zur Herstellung von Fluor-haltigen Verbindungen, insbesondere Organofluorverbindungen dar. Als Perfluoralkylierungsreagenzien werden üblicherweise Perfluoralkylhalogenide, insbesondere Perfluoralkyliodide eingesetzt, die als Quelle von Perfluoralkylradikalen fungieren ("Organofluorine Chemistry. Principles and Commercial Applications." Edited by R.E. Banks, Plenum Press, New York 1994; G.G. Furin, "Some new aspects in the application of perfluoralkyl halides in the synthesis of fluorinecontaining organic compounds" (Review), Russ.Chem.Rev. (English Translation), 69, Nr. 6 (2000), Seiten 491-522; N.O. Brace, "Syntheses with perfluoralkyl iodides. A review, Part III.", J. of Fluorine Chem., 108 (2001), Seiten 147-175; N.O. Brace, "Syntheses with perfluoralkyliodides. Part II", J. of Fluorine Chem. 96 (1999), Seiten 101-127; N.O. Brace, "Syntheses with perfluoralkyl radicals from perfluoralkyl iodides. A rapid survey of synthetic possibilities with emphasis on practical applications. Part one: alkenes, alkynes and allylic compounds", J. of Fluorine Chem., 96 (1999), Seiten 1-25; V.N. Boiko, "Ion-radical perfluoralkylation. Part II.", J. of Fluorine Chem., 69 (1994), Seiten 207-212).

Darüber hinaus werden Perfluoralkylhalogenide zur Herstellung von Perfluoralkyl-, insbesondere Trifluormethyl-Gruppen enthaltenden Organometallverbindungen eingesetzt, die ihrerseits zur Einführung von Perfluoralkylgruppen in organische Moleküle eingesetzt werden können (D.J. Burton, "Fluorinated organometallics: perfluoralkyl and functionalised perfluoralkyl organometallic reagents in organic synthesis", Tetrahedron, 48, Nr. 2 (1992), Seiten 189-275).

Des weiteren wurde das Reagenz TMSCF₃ zur nucleophilen Trifluormethylierung entwickelt (G.K. Surya Prakash, "Nucleophilic trifluormethylation tamed", J. of Fluorine Chem., 112 (2001), Seiten 123-131). Durch die Reaktion von langkettigen Perfluoralkyliodiden mit Tetrakis(dimethylamino)ethylen in Gegenwart von Chlortrimethylsilan wurde dieses Verfahren der nucleophilen Perfluoralkylierung auf weitere organische und anorganische Substrate ausgedehnt (V.A. Petrov, Tetrahedron Letters, 42 (2001), Seiten 3267-3269).

Die vorstehend angegebenen Verfahren zu Perfluoralkylierung haben jedoch den Nachteil, daß die entsprechenden Perfluoralkylhalogenide entweder sehr teuer sind, oder deren Nutzung, wie beispielsweise im Falle der Verbindung CF₃Br, nach dem Montreal Protokoll nur sehr eingeschränkt gestattet ist.

Diese Nachteile führten zu der Entwicklung neuer Peffluoralkylierungsreagenzien, wie sie in J.R. Desmurs et al., 12th European Symposium on Fluorine Chemistry, Berlin, Germany, 1998, Abstracts A23 and A24 beschrieben sind. Die Herstellung dieser Reagenzien gelingt allerdings nur unter Verwendung von CF₃H, einer hochflüchtigen und schwer handhabbaren Verbindung. Ferner wurden weitere stabile Perfluoralkylierungsreagenzien zur nucleophilen Triluormethylierung entwickelt, wobei zur Synthese dieser Reagenzien vom Methylhemiketal des Fluorals ausgegangen wird, welches zuvor in einem relativ aufwendigen Prozeß hergestellt werden muß. Außerdem ist die Anwendung dieser Reagenzien auf die Trifluormethylierung beschränkt (G.Blond et al., Tetrahedron Letters, 42 (2001), Seiten 2437-2475; T. Billard et al., Eur. J. Org. Chem., 2001, Seiten 1467-1471; T. Billard et al. Tetrahedron Leiters, 41 (2000), Seiten 8777-8780; G. Blond et al., J. Org. Chem., 66, Nr. 14 (2001), Seiten 4826-4830).

Der Gegenstand der vorliegenden Erfindung ist daher die Verwendung wenigstens eines Tris(perfluoralkyl)phosphins, zur Perfluoralkylierung von chemischen Substraten.

Zur Perfluoralkylierung von chemischen Substraten mit Perfluoralkylphosphinen ist es erforderlich, das zu perfluoralkylierende Substrat entweder vor oder während der Umsetzung mit dem jeweiligen Perfluoralkylphosphin mit wenigstens einer Base zu behandeln. Bevorzugt erfolgt die Perfluoralkylierung des chemischen Substrates mit wenigstens einem Perfluoralkylphosphin in Gegenwart wenigstens einer Base.

Bevorzugt kommen hierfür starke Basen, wie beispielsweise Kalium tert-Butylat, n-Butyllithium und/oder ein Grignard-Reagenz in Betracht.

Vorzugsweise wird die Perfluoralkylierung in einem geeigneten, ggf. nach üblichen Verfahren getrocknetem Reaktionsmedium, wie beispielsweise cyclischen oder aliphatischen Ether, insbesondere Tetrahydrofuran oder Diethylether, durchgeführt.

Als chemische Substrate kommen bevorzugt organische Verbindungen, insbesondere dreifach koordinierte Organborverbindungen sowie Carbonyl-Gruppen aufweisende organische Verbindungen in Betracht.

Als Organoborverbindungen kommen bevorzugt Tris-(C₁₋₃)-Alkylborate, besonders bevorzugt Trimethylborat zum Einsatz.

Bevorzugte Carbonylgruppen-aufweisende Verbindungen sind ggf. substituierte Diarylketonverbindungen, insbesondere Benzophenon.

Vorzugsweise kann die Perfluralkylierung chemischer Substrate mit Perfluoralkylphosphinen unter Inertgasatmosphäre, wie z.B. Argon oder Stickstoff, durchgeführt werden.

Die Verwendung von Tris(perfluoralkyl)phosphinen als Perfluoralkylierungsreagenzien ist insbesondere deshalb vorteilhaft, weil diese Verbindungen, im Gegensatz zu vielen anderen Perfluoralkylierungsreagenzien, stabile Verbindungen darstellen, was ihre einfache, sichere Handhabung ermöglicht.

Die NMR-Spektren wurden mit Hilfe eines Bruker Avance 300 NMR Spektrometers mit folgenden Frequenzen aufgenommen:
300,1 MHz für ¹H
282,4 MHz für ¹⁹F und
96,3 MHz für ¹¹B.

Die Massenspektren wurden mit einem Gerät vom Typ AMD 604 gemessen.

Im folgenden wird die Erfindung anhand von Beispielen erläutert. Diese Beispiele dienen lediglich der Erläuterung der Erfindung und schränken den allgemeinen Erfindungsgedanken nicht ein.

### Beispiele

### Beispiel 1a:

### Herstellung von Tris(pentafluorethyl)phosphin

In einem FEP(Fluorethylenpolymer)-Kolben wurden 56,0 g (131,4 mmol) Difluortris(pentafluorethyl)phosphoran und 38,0 g (326,8 mmol) Triethylsilan unter heftigem Rühren für 12 Stunden bei einer Badtemperatur von 110°C am Rückfluß erhitzt. Anschließend wurde das Reaktionsgemisch unter Normaldruck unter einer Inertgasatmosphäre destilliert und 48,0 g der Fraktion mit einem Siedebereich von 81-85 °C aufgefangen. Diese Fraktion wurde anschließend auf -20°C abgekühlt und die untere Phase (gewünschtes Produkt) abgetrennt. Es wurden 42,2 g von nahezu reinem Tris(pentafluorethyl)phosphin erhalten, entsprechend einer Ausbeute von 82,8 %, bezogen auf das eingesetzte Difluortris(pentafluorethyl)phosphoran.

Das so erhaltene Produkt wurde mittels ¹⁹F-, ³¹P- und ¹H-NMR-Spektroskopie sowie durch Massenspektroskopie charakterisiert:
¹⁹F NMR Spektrum; δ, ppm:
(Lösungsmittel CDCl₃, interne Referenz CCl₃F)
-82,4 dt (CF₃); -106,5 dq (CF₂); J²_{P,F} = 49,3 Hz; J³_{P,F} = 15,8 Hz; J³_{F,F} = 3,1 Hz

³¹P NMR Spektrum; 6, ppm:
(Lösungsmittel CDCl₃, Referenz 85 Gew.-%ige H₃PO₄)
13,3 sep of dec

Die Werte der gefundenen chemischen Verschiebungen entsprechen den aus der Veröffentlichung von J.J. Kampa et al., Angew. Chem., 107, Nr. 11 (1995), Seiten 1334-1337 bekannten Werten.

Hochauflösendes Massenspektrum:
Berechnet (M+): 387,949812
Gefunden (M+): 387,949842

### Beispiel 1b:

### Herstellung von Tris(pentafluorethyl)phosphin

In einem Glaskolben wurden 230,0 g (0,54 mol) Difluortris(pentafluorethyl)phosphoran und 41,2 g (1,089 mol) Natriumborhydrid unter heftigem Rühren für 3 Stunden bei einer Badtemperatur von ca. 110 °C unter Rückfluß erhitzt. Anschließend wurde das Reaktionsgemisch unter vermindertem Druck (2 KPa) destilliert und zwei Fraktionen des Produkts in Kühlfallen mit einer Temperatur von -78°C bzw. - 195 °C aufgefangen. Zur weiteren Reinigung wurden die vereinigten Produkte von beiden Fällen bei Normaldruck unter Inertgas-Atmosphäre destilliert, wobei die Fraktion im Siedebereich 85-87 °C aufgefangen wurde. Es wurden 194,0 g reines Tris(pentafluorethyl)phosphin erhalten. Die Ausbeute betrug 93 %, bezogen auf die Menge an eingesetztem Difluortris(pentafluorethy)-phosphoran.

Das so erhaltene Produkt wurde mittels ¹⁹F- und ³¹P-NMR-Spektroskopie charakterisiert. Die gefundenen chemischen Verschiebungen entsprechen denen aus Beispiel 1a.

### Beispiel 2:

### Perfluoralkylierung von Benzophenon

5,81 g (14,97 mmol) Tris(pentafluorethyl)phosphin wurden unter Inertgas-Atmosphäre zu einer Lösung von 1,68 g (14,97 mmol) Kalium-tert.-Butylat und 2,72 (14,93 mmol) Benzophenon in 20 cm³ getrocknetem Tetrahydrofuran unter Kühlung mit einem Wasserbad so zugegeben, daß die Temperatur des Reaktionsgemisches bei ca. 20°C lag. Anschließend wurde die Reaktionsmischung eine Stunde bei Raumtemperatur nachgerührt, mit 20 cm³ einer 0,1 N Lösung von HCl versetzt und zweimal mit jeweils 75 cm³ Dietehylether extrahiert. Die vereinigten Extrakte wurden dreimal mit jeweils 20 cm³ Wasser gewaschen und über wasserfreiem Magnesiumsulfat getrocknet. Das Lösungsmittel wurde anschließend abdestilliert und das gewünschte Produkt aus n-Hexan auskristallisiert. Es wurden 2,8 g
2,2,3,3,3-Pentafluor-1,1-diphenylpropan-1-ol mit einem Schmelzpunkt im Bereich von 82-83 °C erhalten.

Die Ausbeute betrug 62 %, bezogen auf die eingesetzte Menge an Benzophenon.

Das so erhaltene Produkt wurde mittels ¹⁹F- und ¹H-NMR-Spektroskopie sowie durch hochauflösende Massenspektroskopie und Elementaranalyse charakterisiert:
¹⁹F NMR Spektrum; δ, ppm:
(Lösungsmittel CDCl₃, interne Referenz CCl₃F)
-77,6 s (CF₃); -116,9 m (CF₂);

¹H-NMR Spektrum; δ, ppm:
(Lösungsmittel CDCl₃, Referenz TMS)
7,53-7,67 m (2H), 7,30-7,47 m (3H), 2,85 br.s (OH)

Der Schmelzpunkt sowie die gefundenen chemischen Verschiebungen stimmen mit den aus der Veröffentlichung von L.S. Chen et al., J. of Fluorine Chem., 20 (1982), Seiten 341-348 bekannten Werten überein.

Hochauflösendes Massenspektrum:
Berechnet (M+): 302,073006
Gefunden (M+): 302,073115

Elementaranalyse:
Gefunden; C 59,67 %; H 3,62 %
Berechnet für (C₆H₅)₂C(OH)C₂F₅: C 59,61 %; H 3,67 %

### Beispiel 3:

### Kaliumpentafluorethyltrifluorborat (C₂F₅)BF₃K

### a)

9,78 g (25,21 mmol) Tris(pentafluorethyl)phosphin wurden langsam unter Inertgas-Atmosphäre zu einer gerührten Lösung von 30 mmol n-Butyllithium (15 cm³ einer 2 M Lösung in Cyclohexan) in 70 cm³ getrocknetem Diethylether unter Kühlung so zugegeben, daß die Temperatur des Reaktionsgemisches bei ca. - 60 °C lag. Anschließend wurde die Reaktionsmischung 30 Minuten bei - 55°C nachgerührt und eine Lösung von 3,31 g (31,85 mmol) Trimethylborat (CH₃O)₃B in 5 cm³ getrocknetem Diethylether zugegeben.

Die so erhaltene Reaktionsmischung wurde auf Raumtemperatur gebracht und mit 30 cm³ Flusssäure (48 %ige wäßrige Lösung) versetzt und anschließend weitere 10 Stunden bei Raumtemperatur gerührt. Im Anschluß daran wurden 10,0 g (128 mmol) Kaliumhydrogendifluorid zugegeben und weitere 10 Stunden bei Raumtemperatur gerührt. Die organische Phase wurde abgetrennt, durch Rühren über festem Kaliumcarbonat neutralisiert und mit wasserfreiem Magnesiumsulfat getrocknet. Das Lösungsmittel wurde anschließend abdestilliert und der so erhaltene Rückstand in einer möglichst geringen Menge Tetrahydrofuran gelöst. Durch Zugabe von Chloroform wurde dann ein Feststoff ausgefällt, der abfiltriert und getrocknet wurde. Es wurden 3,30 g Kaliumpentafluorethyltrifluorborat in Form eines weißen Feststoffes erhalten. Die Ausbeute betrug 58 %, bezogen auf das eingesetzte Tris(pentafluorethyl)phosphin.

Das so erhaltene Produkt wurde mittels ¹¹B- und ¹⁹F-NMR-Spektroskopie sowie durch Elementaranalyse charakterisiert:
¹¹B-NMR Spektrum; δ, ppm:
(Lösungsmittel Aceton-D6, externe Referenz BF₃ O(C₂H₅)₂):
0,2 tq; J¹_{B,F} = 41,0 Hz; J²_{B,F} = 20,0 Hz

¹⁹F NMR Spektrum; δ, ppm:
(Lösungsmittel Aceton-D6, interne Referenz CCl₃F)
-83,3 q (CF₃); -136,2 q, (CF₂); -153,5 q (BF₃); J¹_{B,F} = 41,2 Hz; J²_{B,F} =19,9 Hz; J⁴_{F,F} = 4,9 Hz.

Elementaranalyse:
Gefunden: C 10,56 %
Berechnet (für (C₂F₅)BF₃K): C 10,63 %

### b)

### Kaliumpentafluorethyltrifluorborat (C₂F₅)BF₃K

20,81 g (53,63 mmol) Tris(pentafluorethyl)phosphin wurden unter Inertgasatmosphäre langsam zu einer gerührten Lösung von 60 mmol Methylmagnesiumchlorid (20 cm³ einer 3 M Lösung in Tetrahydrofuran) in 100 cm³ getrocknetem Tetrahydrofuran unter Kühlung so zugegeben, daß die Temperatur des Reaktionsgemisches bei ca. -60 °C lag. Anschließend wurde die Reaktionsmischung 30 Minuten bei - 55 °C nachgerührt und 6,24 g (60,04 mmol) Trimethylborat (CH₃O)₃B zugegeben. Die so erhaltene Reaktionsmischung wurde auf Raumtemperatur gebracht und mit 25 cm³ Flusssäure (48 %ige wäßrige Lösung) versetzt und anschließend weitere 10 Stunden bei Raumtemperatur gerührt.

Im Anschluß daran wurden 15,0 g (192 mmol) Kaliumhydrogendifluorid zugegeben und weitere 10 Stunden bei Raumtemperatur gerührt. Die organische Phase wurde abgetrennt, durch Rühren über festem Kaliumcarbonat neutralisiert und mit wasserfreiem Magnesiumsulfat getrocknet. Das Lösungsmittel wurde anschließend abdestilliert und der so erhaltene Rückstand in einer möglichst geringen Menge Tetrahydrofuran gelöst. Durch Zugabe von Chloroform wurde dann ein Feststoff ausgefällt, der abfiltriert und getrocknet wurde. Es wurden 6,30 g Kaliumpentafluorethyltrifluorborat in Form eines weißen Feststoffes erhalten. Die Ausbeute betrug 52 %, bezogen auf das eingesetzte Tris(pentafluorethyl)phosphin.

Das so erhaltene Produkt wurde mittels ¹¹B- und ¹⁹F-NMR-Spektroskopie charakterisiert. Die entsprechenden Signale stimmten mit den unter 5a genannten Signalen überein.

## Patentansprüche

1. Verwendung wenigstens eines Tris(perfluoralkyl)phosphins zur Perfluoralkylierung chemischer Substrate.

2. Verwendung gemäß Anspruch 1, **dadurch gekennzeichnet, daß** die Perfluoralkylierung in Gegenwart einer Base durchgeführt wird.

3. Verwendung gemäß Anspruch 1 oder 2, **dadurch gekennzeichnet, daß** als Substrate organische Verbindungen eingesetzt werden.

4. Verwendung gemäß Anspruch 1, 2 oder 3, **dadurch gekennzeichnet, dass** als Substrate dreifach koordinierte Organoborverbindungen und/oder Carbonyl-Gruppen-haltige organische Verbindungen eingesetzt werden.

## Claims

1. Use of at least one tris(perfluoroalkyl)phosphine for the perfluoroalkylation of chemical substrates.

2. Use according to Claim 1, **characterised in that** the perfluoroalkylation is carried out in the presence of a base.

3. Use according to Claim 1 or 2, **characterised in that** the substrates employed are organic compounds.

4. Use according to Claim 1, 2 or 3, **characterised in that** the substrates employed are tricoordinated organoboron compounds and/or organic compounds containing carbonyl groups.

## Revendications

1. Utilisation d'au moins une tris(perfluoroalkyl)phosphine pour la perfluoroalkylation de substrats chimiques.

2. Utilisation selon la revendication 1, **caractérisée en ce que** la perfluoroalkylation est effectuée en présence d'une base.

3. Utilisation selon la revendication 1 ou 2, **caractérisée en ce que** les substrats employés sont des composés organiques.

4. Utilisation selon la revendication 1, 2 ou 3, **caractérisée en ce que** les substrats employés sont des composés de bore organique tricoordonné et/ou des composés organiques contenant des groupements carbonyle.
